# FASCICULE DE BREVET EUROPEEN

(11) **EP 0 360 717 B1**
(45) Date de publication et mention de la délivrance du brevet: **14.04.1993**
(21) Numéro de dépôt: 89440096.9
(22) Date de dépôt: 18.09.1989
(51) Int. Cl.: A61M 39/00

(54) **Cathéter tournable par le raccord**
Katheter, der beim Anschluss drehbar ist
Catheter rotatable upon connection

(30) Priorité: 22.09.1988 FR 8812564
(43) Date de publication de la demande: 28.03.1990
(73) Titulaire: MEDICORP RESEARCH LABORATORIES CORPORATION, Arlington, Virginia 22203 (US)
(72) Inventeur: Amor, Max, Dr., F-54500 Vandoeuvre (FR); Karcher, Gilles, Dr., F-54000 Nancy (FR); Ethevenot, Gérard, Dr., F-54000 Nancy (FR)
(74) Mandataire: Aubertin, François

(56) Documents cités:
- EP-A- 0 151 519
- DE-A- 2 907 832
- GB-A- 1 193 759
- US-A- 4 254 773
- US-A- 4 433 973

## Description

L'invention concerne un cathéter à usage selon le préambule de la revendication 1. Un cathéter de ce type est décrit dans US-A-4254773.

Cette invention trouvera son application dans le domaine médical et concerne, plus particulièrement, les industriels spécialisés dans la conception d'ustensiles destinés, spécifiquement, à un usage dans ce domaine.

Les cathéters sont des appareils de plus en plus utilisés pour procéder, par voie percutanée, soit à des traitements particuliers de certains organes, soit, simplement, pour explorer ces derniers.

L'une des maladies soignée au moyen de ces cathéters est, par exemple, l'artérite. Cette maladie consiste, en fait, en un rétrécissement des vaisseaux d'origine athéromateuse pouvant l'obturer partiellement, il s'agit, dans ce cas, d'une sténose ou complètement, la trombose définissant cet état.

Dans de nombreux cas d'artérite, on procède à la déobstruction artérielle par voie percutanée au moyen d'un cathéter à balonnet pour forcer la sténose à s'ouvrir.

Par ailleurs, l'angiographie, qui consiste en une radiographie des vaisseaux sanguins, nécessite, fréquemment, l'utilisation de cathéters, pour injecter, dans les veines ou les artères d'une région donnée, une substance opaque permettant de visualiser lesdits vaisseaux aux rayons X.

Dans tous les cas d'utilisation, le cathéter se compose d'un tube principal, muni d'une extrémité distale de forme prédéterminée en fonction des cavités à atteindre. Ce tube principal comporte, en outre, une extrémité proximale pourvue de moyens de connexion autorisant le raccordement du cathéter à un appareillage externe, tel qu'une rampe à robinet ou un cordon de raccordement intermédiaire ou autre.

Plus précisément, ces moyens de connexion se composent d'un corps cylindrique présentant un alésage central ajusté au diamètre externe du tube principal du cathéter afin de faciliter l'engagement de l'extrémité proximale de ce dernier.

Ce corps cylindrique est rendu solidaire dudit tube principal, soit par surmoulage, soit par collage, soit par un procédé au solvant. Il est, par ailleurs, prolongé, à son extrémité libre, par un manchon de connexion du type universel.

Ainsi, ce manchon de connexion est de forme cylindrique et comporte un canal coaxial présentant une légère conicité à son extrémité intérieure tentant à réduire sa section. Ce canal débouche dans l'alésage du corps principal donc dans le cathéter et sert, plus précisément, à accueillir l'embout de moyens de raccordement conjugués associé audit appareillage externe. Ledit manchon de connexion comporte, en outre, à son extrémité libre et sur son pourtour externe des ergots susceptibles de coopérer avec un filetage présent au niveau desdits moyens de raccordement conjugués.

Au vu de la description ci-dessus d'un cathéter correspondant à l'état antérieur de la technique, on constate qu'une fois raccordé à un appareillage ou à une conduite externe, la mobilité de ce cathéter est strictement dépendante de la mobilité de cet appareillage ou de ladite conduite externe.

Or, il est bien évident qu'au cours de sa progression dans le corps du patient, il doit être possible d'orienter aisément l'extrémité distale du cathéter de manière à atteindre l'organe qu'il convient d'ausculter ou de traiter. Cette orientation ne peut être obtenue qu'en imprimant, un déplacement axial ou une rotation à la partie externe du cathéter. A cet effet, il y a lieu de noter que le tube principal est généralement rigidifié, soit en raison de la présence d'un mandrin en fil introduit dans son logement intérieur, soit au moyen d'une structure tressée noyée dans l'épaisseur dudit tube principal.

Toutefois, au cas où les appareillages externes ou la conduite associée au cathéter sont relativement encombrants, celui-ci est, nécessairement, d'une maniabilité limitée et, dans tous les cas, peu aisée. Sans compter que, selon le sens de rotation conféré au cathéter, une moindre résistance au niveau des appareillages ou de la conduite accompagnant son mouvement peut provoquer une déconnexion à un endroit quelconque du réseau et, finalement, altérer l'opération ou la mesure en cours.

Dans ce but, il a été conçu des raccords tels que décrits dans le document US A-4.254.773 dont la caractéristique essentielle consiste en ce qu'ils autorisent la rotation des conduites ou des éléments qu'ils raccordent l'un par rapport à l'autre. Aussi, en intercalant un tel raccord rotatif entre les moyens de connexion du cathéter et la conduite ou l'appareillage externe, l'opérateur est en mesure de provoquer la rotation dudit cathéter sans pour autant imprimer à cet appareillage ou à cette conduite externe, un mouvement identique.

Toutefois, les inconvénients que constituent ces raccords rotatifs consistent, d'une part, en ce qu'ils représentent un élément supplémentaire dont il faut garantir l'approvisionnement et le stockage au niveau de l'établissement hospitalier. Sans compter qu'ils contribuent à un surcroît de dépenses.

D'autre part, ce raccord rotatif présente l'inconvénient qu'il n'apporte aucune solution aux éventuelles déconnexions possibles telles qu'évoquées ci-dessus. En effet, quelle que soit la partie manipulée par l'opérateur, à savoir le raccord rotatif ou le cathéter, le mouvement se transmet au travers d'au moins une connexion, celle séparant ledit cathéter et le raccord rotatif. Par ailleurs, on notera que la proximité de ces derniers et des moyens de raccordement du cathéter peut amener l'opérateur à procéder involontairement, à une fausse manoeuvre, et, finalement, provoquer cette déconnexion.

La présente invention a pour but de remédier à l'ensemble des inconvénients précités en proposant un cathéter qui soit muni de moyens appropriés permettant de lui imprimer une rotation sans que cette dernière soit répercutée sur un appareillage ou une conduite externe au travers de l'une quelconque des connexions du réseau.

Le problème est résolu par les caractéristiques de la seconde partie de la revendication 1.

De ce qui précède, il en résulte, bien évidemment, l'avantage que les risques de déconnexion inopinée sont annihilés et que l'on peut s'abstenir de l'utilisation et de l'approvisionnement d'un élément subsidiaire.

L'invention est exposée ci-après plus en détail à l'aide de dessins représentant seulement un mode d'exécution.
- La figure 1 représente une vue schématisée et en plan d'un cathéter conforme à l'invention.
- la figure 2 représente une vue en coupe selon II-II de la figure 1.

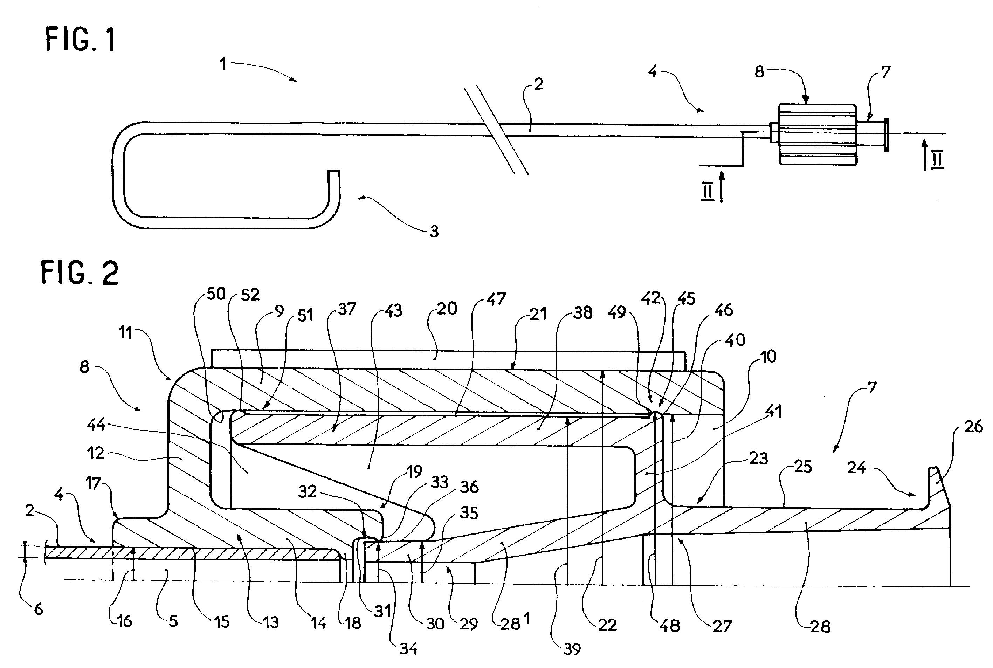

La présente invention est relative à un cathéter dont un mode de réalisation particulier est illustré dans la figure 1.

Ce cathéter est, en fait, à usage médical, et destiné, notamment, à être introduit dans une partie du corps d'un individu par exemple pour procéder à une endoscopie ou encore pour injecter un liquide de diagnostic ou à fonction thérapeutique au niveau d'un organe spécifique. Bien évidemment, on comprendra que le cathéter, objet de la présente invention, n'est nullement limité aux utilisations évoquées ci-dessus. En effet, il existe, à ce jour, beaucoup d'autres techniques thérapeutiques ou de diagnostic dans lesquels interviennent ces cathéters.

Toutefois, quelle que soit la fonction qui lui est attribuée, un cathéter 1 comporte un tube principal 2 de longueur et de diamètre variable et pourvu d'une extrémité distale 3 de forme prédéterminée selon l'organe qu'il convient d'atteindre afin de l'explorer ou d'un assurer le traitement.

Ce tube principal 2 est, généralement, de nature souple ou semi-rigide, de manière à s'accomoder aisément à la forme et aux dimensions des passages qu'il est amené à emprunter. Aussi, les matériaux communément utilisés pour sa conception sont les thermo-plastiques à qualité médicale. Les plus fréquemment rencontrés sont le polyuréthane et le polyéthylène en raison de leur caractère inerte au vu des agents véhiculés et aux substances physiologiques avec lesquels le cathéter est amené en contact. Il peut encore être conçu en métal résistant à la corrosion par le sang, les solutions salines et toute autre substance physiologique ou de nature thérapeutique.

En cas de conception à partir d'un thermoplastique, le tube principal 2 est, habituellement, rigidifié de manière à faciliter son orientation depuis l'extrémité proximale 4, externe au patient. Deux techniques sont adoptées à ce jour :
- soit on emploie un mandrin en fil introduit dans le logement interne 5 du cathéter 1, lors de sa mise en place dans le corps du patient, puis retiré en cours d'examen ou de traitement ;
- soit on intègre, dans l'épaisseur 6 du tube principal 2, selon des techniques connues, une structure tressée, dans ce cas, on s'assurera, toutefois, que l'extrémité distale 3 conserve intégralement sa souplesse afin de ne pas blesser des organes rencontrés.

Par ailleurs, ledit cathéter 1 et, notamment, le tube principal 2 comporte, à son extrémité proximale 4, des moyens de connexion 7 de type universel autorisant son raccordement à un appareillage externe, tel qu'une conduite de transition, une rampe à robinets ou autre.

A cette extrémité proximale 4 du tube principal 2 sont également associés, conformément à l'invention, les moyens de manoeuvre 8 permettant de commander en rotation au moins ledit tube principal 2 par rapport auxdits moyens de connexion 7.

En fait, jusqu'à présent, cette possibilité de manipuler aisément, le cathéter 1 n'était due qu'à des moyens dissociés et, plus précisément, des raccords rotatifs. Cependant, ceux-ci présentent l'inconvénient d'être peu sûrs en raison des risques de déconnexion qu'ils procurent. De plus, ils constituent un élément supplémentaire dont il convient d'assurer l'approvisionnement et le stockage dans les lieux d'utilisation.

En associant, directement, des moyens de manoeuvre 8 au cathéter 1, la présente invention remédie à ces inconvénients.

Préférentiellement, lesdits moyens de manoeuvre 8 sont directement solidaires, d'une part, du tube principal 2 et, d'autre part, des moyens de connexion 7.

Ainsi, selon un mode de réalisation préférentiel, ces moyens de manoeuvre 8 comportent un corps 9 de type tubulaire, présentant un alésage central 10 et muni, à l'une de ses extrémités 11, d'un flasque annulaire 12. Celui-ci maintient, en son centre, un manchon 13 dont une partie 14 s'étend, sensiblement, dans l'alésage central 10 du corps 9. Ce manchon 13 présente un alésage 15 situé dans le prolongement axial dudit alésage central 10 et ajusté au diamètre externe 16 du tube principal 2.

De ce fait, lors de l'assemblage du cathéter 1, l'extrémité proximale 4 dudit tube principal 2 peut être introduite, depuis l'extrémité externe 17 du manchon 13 dans l'alésage 15 jusqu'à venir en butée contre un rebord périphérique interne 18 aménagé sur la paroi interne de ce dernier et, plus précisément, à proximité de l'extrémité 19 de la partie 14 introduite dans l'alésage central 10. Puis, le manchon 13 est rendu solidaire du tube principal 2, soit par collage, soit par surmoulage ou encore selon un procédé au solvant au cas où les matériaux utilisés l'autorise. A ce sujet, il convient de noter que ces moyens de manoeuvre 8 peuvent être conçus en un matériau identique à ceux constituant le tube principal et, de préférence, en un thermoplastique tel que le polyuréthane ou le polyéthylène.

Ainsi, après assemblage, le mouvement imprimé du corps 9 des moyens de manoeuvre 8 est directement répercuté sur le tube principal 2 du cathéter 1. On notera que, pour faciliter la préhension du corps 9, celui-ci peut présenter une section polygonale ou encore comporter une série d'arêtes 20 réparties sur son pourtour externe de forme cylindrique 21 et disposées selon des génératrices. Par ailleurs, le diamètre 22 dudit corps 9 sera déterminé de sorte que la commande au moyen de deux doigts, soit en mesure d'exercer un couple suffisant sur le cathéter 1 afin d'en garantir la rotation.

Quant aux moyens de connexion 7, ils comportent un embout 23, formé d'un tronçon cylindrique 28 et d'un tronçon tronconique 28A. Plus précisément, le tronçon cylindrique 28 comporte à son extrémité libre 24, et sur son pourtour externe 25 des ergots 26 destinés à coopérer avec le filetage des moyens de raccordement d'un appareillage ou d'une conduite externe non représentée. Quant au tronçon tronconique 28A, prolongeant le tronçon cylindrique 28, il présente à son extrémité effilée 29, une portion cylindrique 30. Celle-ci est, plus précisément, destinée à être introduite, partiellement, et au niveau de l'extrémité interne 19, dans le manchon 13 des moyens de manoeuvre 8. Aussi, l'alésage 15 dudit manchon 13 comporte-t-il, au-delà du rebord périphérique interne 18 servant de butée au tube principal 2, un décrochement 31 de diamètre légèrement supérieur au diamètre externe 35 de la portion cylindrique 30. Cet alésage 15 est muni, en outre, au niveau de son extrémité 32, débouchant dans l'alésage central 10, d'un bossage interne 33 réduisant son diamètre 34 de manière à l'ajuster ou, préférentiellement, à le rendre légèrement inférieur au diamètre externe 35 de la portion cylindrique 30. Cette disposition impose l'introduction, légèrement en force, de cette portion cylindrique 30 dans le manchon 13 pour provoquer l'effacement du bossage interne 33 par effet d'élasticité de la matière thermo-plastique. Finalement, ledit bossage interne 33 vient à coopérer avec une certaine pression avec le pourtour externe 36 de la portion cylindrique 30 et garantit l'étanchéité entre les moyens de manoeuvre 8 et les moyens de connexion 7.

Ceux-ci comportent, en outre, des moyens de rotation 37 par rapport auxdits moyens de manoeuvre 8 consistant en une douille 38 venant s'emboîter dans l'alésage central 10 et, à cet effet, présentant un diamètre externe 39 légèrement inférieur au diamètre interne 40 de ce dernier. Cette douille 38 est rendue solidaire de l'embout 23, d'une part, au moyen d'un flasque annulaire 41 situé à son extrémité 42 orientée en direction du tronçon cylindrique 28 et, d'autre part, par l'intermédiaire de nervures de renfort 43. Celles-ci sont en nombre variable réparties dans la partie interne 44 de la douille 38 et coopérant, plus précisément, avec le tronçon tronconique 28A de l'embout 23.

Cette douille 38 comporte, en outre, des moyens 45 pour maintenir les moyens de connexion 7 emmanchés dans les moyens de manoeuvre 8. Préférentiellement, de tels moyens 45 consistent en un bourrelet périphérique 46 garnissant le pourtour externe 47 de la douille 38 au niveau de son extrémité 42. Ce bourrelet périphérique 46 présente un diamètre 48 légèrement supérieur au diamètre interne 40 de l'alésage central 10 du corps 9 de sorte qu'après engagement, avec une légère pression, des moyens de connexion 7 dans les moyens de manoeuvre 8, ledit bourrelet périphérique 46 soit en mesure de s'engager dans une gorge 49 usinée dans la paroi interne 50 du corps 9. Ainsi, ce bourrelet périphérique 46 permet d'obtenir un blocage en translation des moyens de connexion 7 dans les moyens de manoeuvre 8 et, par là même, un parfait guidage en rotation de la douille 38 dans l'alésage central 10 du corps 9.

Avantageusement, on retrouve l'extrémité libre 51 de ladite douille 38 et sur son pourtour externe 47, un bossage 52 coopérant avec un jeu réduit, avec la paroi interne 50 du corps 9 de manière à contribuer à ce guidage en rotation, dans des conditions idéales, des moyens de connexion 7 par rapport aux moyens de manoeuvre 8.

Au vu de la description qui précède, il est à considérer que la présente invention tout en étant d'une complexité réduite, apporte de sérieux avantages aux cathéters à usage médical. D'une part, elle leur confère une parfaite maniabilité tout en réduisant, d'autre part, les risques de déconnexion connus jusqu'ici.

## Revendications

1. Cathéter à usage médical, destiné à être introduit dans le corps d'un individu pour l'endoscopie ou l'injection de liquide de diagnostic ou à fonction thérapeutique, comportant un tube principal (2), muni d'une extrémité distale (3) et d'une extrémité proximale (4), celle-ci étant pourvue de moyens de connexion (7) à un appareillage externe, comportant au niveau de l'extrémité proximale (4) des moyens de manoeuvres (8), reliés, par l'intermédiaire de moyens de rotation (37) aux moyens de connexion (7), pour commander en rotation au moins ce tube principal (2) par rapport à ces moyens de connexion (7), caractérisé par le fait que les moyens de manoeuvre (8) sont rendus solidaires directement, sans pièce de connexion intermédiaire du tube principal (2).

2. Cathéter selon la revendication 1, caractérisé par le fait que les moyens de manoeuvre (8) comportent un corps (9) pourvu d'un alésage central (10) et muni, à l'une de ses extrémités (11), d'un flasque annulaire (12) maintenant, en son centre, un manchon (13) dont une partie (14) s'étend, sensiblement, à l'intérieur dudit alésage central (10) du corps 9.

3. Cathéter selon la revendication 2, caractérisé par le fait que le manchon (13) comporte un alésage (15) situé dans le prolongement axial de l'alésage central (10) du corps (9) et ajusté au diamètre externe (16) du tube principal (2), dans cet alésage (15) étant introduite l'extrémité proximale (4) de ce dernier pour rendre solidaires les moyens de manoeuvre (8) du cathéter (1), soit par surmoulage, soit par collage ou selon un procédé au solvant.

4. Cathéter selon la revendication 3, caractérisé par le fait que l'alésage (15) du manchon (13) présente sur sa paroi interne un rebord périphérique interne (18) contre lequel vient buter l'extrémité proximale (4) du tube principal (2), une fois introduit dans ledit alésage (15).

5. Cathéter selon la revendication 2, caractérisé par le fait que le corps (9) des moyens de manoeuvre (8) est de section polygonale ou cylindrique et comporte, sur son pourtour externe (21) et disposées selon des génératrices, des arêtes (20) pour faciliter la transmission du couple, exercée par l'opérateur, sur le cathéter (1).

6. Cathéter selon les revendications 1 et 3, caractérisé par le fait que les moyens de connexion (7) sont du type universel et sont constitués par un embout (23) formé d'un tronçon cylindrique (28) et d'un tronçon tronconique (28A) situés dans le prolongement axial l'un de l'autre, le tronçon cylindrique (28) comportant, à son extrémité libre (24) et sur son pourtour externe (25), des ergots (26) destinés à coopérer avec le filetage des moyens de raccordement d'un appareillage externe, une portion cylindrique (30) susceptible de coopérer avec le manchon (13) des moyens de manoeuvre (8), aboutant à l'extrémité effilée (29) du tronçon tronconique (28A).

7. Cathéter selon les revendications 4 et 6, caractérisé par le fait que l'alésage (15) dans le manchon (13) présente, au niveau de l'extrémité (19) de ce dernier, interne à l'alésage central (10) du corps (9), et au-delà du rebord périphérique interne (18), un décrochement (31) de diamètre légèrement supérieur au diamètre externe (35) de la portion cylindrique (30) pour en faciliter l'introduction, l'alésage (15) étant muni, en outre, à son extrémité (32) débouchant dans l'alésage central (10) d'un bossage interne (33) susceptible de coopérer avec une certaine pression avec le pourtour externe (36) de ladite portion cylindrique (30) et garantir l'étanchéité entre les moyens de manoeuvre (8) et les moyens de connexion (7).

8. Cathéter selon les revendications 1 et 6, caractérisé par le fait que les moyens de rotation (37) sont formés par une douille (38) de diamètre externe (39) légèrement inférieur au diamètre interne (30) de l'alésage central (10) du corps (9) pour autoriser son engagement dans ce dernier, ladite douille (38) étant rendue solidaire de l'embout (23), d'une part, au moyen d'un flasque annulaire (41) situé à son extrémité (42) orientée en direction du tronçon cylindrique (28) et, d'autre part, par l'intermédiaire de nervures de renfort (43) disposées dans sa partie interne (44) et coopérant avec le tronçon tronconique (28A).

9. Cathéter selon la revendication 8, caractérisé par le fait que la douille (38) comporte, d'une part, des moyens (45) pour maintenir les moyens de connexion (7) emmanchés dans les moyens de manoeuvre (8), ces moyens (45) étant constitués par un bourrelet périphérique (46) garnissant le pourtour externe (47) au niveau de l'extrémité (42) de ladite douille (38) et, venant s'engager dans une gorge (49) usinée dans la paroi interne (50) du corps (9) et, d'autre part, un bossage (52) aménagé à son extrémité libre (51) et coopérant avec un certain jeu, avec ladite paroi interne (50) du corps (9), pour contribuer au guidage en rotation des moyens de manoeuvre (8) par rapport aux moyens de connexion (7).

## Claims

1. Catheter for medical use, intended for being introduced into the body of an individual for the purpose of endoscopy or inejction of a liquid for diagnostics or with a therapeutical function, including a main pipe (2) provided with a distal end (3) and a proximal end (4), this latter being provided with means (7) for the connection to an external equipment, including, at the level of the proximal end (4), operating means (8), connected through rotating means (37) to the connecting means (7), for controlling the rotation of at least this main pipe (2) with respect to these connecting means (7), characterized in that the operating means (8) are directly made integral, without any intermediate connecting part, with the main pipe (2).

2. Catheter according to claim 1, characterized in that the operating means (8) include a body (9) provided with a central bore (10) and, at its one end (11), with an annular side plate (12) keeping, in its centre, a sleeve (13) a portion (14) of which substantially extends inside said central bore (10) of the body (9).

3. Catheter according to claim 2, characterized in that the sleeve (13) includes a bore (15) located in the axial extension of the central bore (10) of the body (9) and adjusted to the outer diameter (16) of the main tube (2), into this bore (15) being engaged the proximal end (4) of this latter in order to make the operating means (8) integral with the catheter (1) either by moulding or by bonding or by a dissolvent-based process.

4. Catheter according to claim 3, characterized in that the bore (15) of the sleeve (13) has, on its inner wall, an internal peripheral raised edge (18) against which abuts the proximal end (4) of the main pipe (2) once it is introduced into said bore (15).

5. Catheter according to claim 2, characterized in that the body (9) of the operating means (8) has a polygonal or cylindrical cross-section and includes, at its outer periphery (21) and arranged according to generating lines, edges (20) in order to facilitate the transmission of the torque exerted by the operator onto the catheter (1).

6. Catheter according to claims 1 and 3, characterized in that the connecting means (7) are universal-type ones and are comprised of a nipple (23) formed of a cylindrical length (28) and a truncated length (28A) located in the axial extension of each other, the cylindrical length (28) including, at its free end (24) and on its outer periphery (25), catches (26) intended for co-operating with the thread of the connecting means of an external equipment, a cylindrical portion (30) capable of co-operating with the sleeve (13) of the operating means (8) abutting against the tapered end (29) of the truncated length (28A).

7. Catheter according to claims 4 and 6, characterized in that the bore (15) in the sleeve (13) has, at the level of the end (19) of same, inside the central bore (10) of the body (9) and beyond the inner peripheral raised edge (18), a set-back (31) with a diameter slightly larger than the outer diameter (35) of the cylindrical portion (30) in order to facilitate the introduction, the bore (15) being furthermore provided, at its end (32) ending into the cenral bore (10), with an internal shoulder (33) capable of co-operating with some pressure with the outer periphery (36) of said cylindrical portion (30) and ensuring the tightness between the operating means (8) and the connecting means (7).

8. Catheter according to claims 1 and 6, characterized in that the rotating means (37) are formed by a bushing (38) with an outer diameter (39) slightly smaller than the inner diameter (30) of the central bore (10) of the body (9) in order to authorize its engagement into this latter, said bushing (38) being made integral with the nipple (23), on the one hand, by means of an annular side plate (41) located at its end (42) oriented towards the cylindrical length (28) and, on the other hand, through reinforcing ribs (43) arranged in its inner portion (44) and co-operating with the truncated length (28A).

9. Catheter according to claim 8, characterized in that the bushing (38) includes, on the one hand, means (45) for maintaining the connecting means (7) fixed into the operating means (8), these means (8) being formed by a peripheral rim (46) the outer periphery (47) is fitted with at the level of the end (42) of said bushing (38) and engaging into a groove (49) made in the inner wall (40) of the body (9) and, on the other hand, a shoulder (52) made at its free end (51) and co-operating with some backlash with said inner wall (50) of the body (9) in order to contribute to the guiding of the operating means (8) while rotating with respect to the connecting means (7).

## Patentansprüche

1. Katheter für medizinische Verwendung, der dazu bestimmt ist, für die Endoskopie oder zum Einspritzen einer Diagnosenflüssigkeit oder einer Flüssigkeit mit einer therapeutischen Funktion in den Körper eines Menschens hineingeführt zu werden und ein Hauptrohr (2) umfaßt, das mit einem distalen Ende (3) und einem proximalen Ende (4) versehen ist, wobei dieses letzte mit Mitteln (7) für den Anschluß an ein externes Gerät versehen ist und der im Bereich des proximalen Ende (4) Betätigungsmittel (8) umfaßt, die zum Steuern der Drehbewegung von wenigstens diesem Hauptrohr (2) bezüglich dieser Anschlußmittel (7) über Drehmittel (37) mit den Anschlußmittel (7) verbunden sind, dadurch gekennzeichnet, daß die Betätigungsmittel (8) direkt, ohne Zwischenanschlußteil, mit dem Hauptrohr (2) fest verbunden ist.

2. Katheter nach Anspruch 1, dadurch gekennzeichnet, daß die Betätigungsmittel (8) einen Körper (9) umfassen, der mit einer Zentralbohrung (10) und an seinem einen Ende (11) mit einer ringförmigen Seitenplatte (12) versehen ist, die in deren Mitte eine Muffe (13) hält, wovon sich ein Teil (14) im wesentlichen innerhalb der genannten Zentralbohrung (10) des Körpers (9) erstreckt.

3. Katheter nach Anspruch 2, dadurch gekennzeichnet, daß die Muffe (13) eine Bohrung (15) umfaßt, die sich in der axialen Verlängerung der Zentralbohrung (10) des Körpers (9) befindet und dem Außendurchmesser (16) des Hauptrohres (2) angepaßt ist, wobei das proximale Ende (4) dieses letzten in diese Bohrung (15) hineingeführt wird, um die Betätigungsmittel (8) entweder durch Abformung oder durch Anleimung oder mittels eines Lösungsmittelverfahrens fest mit dem Katheter (1) zu verbinden.

4. Katheter nach Anspruch 3, dadurch gekennzeichnet, daß die Bohrung (15) der Muffe (13) an ihrer Innenwand einen Innenumkreisrand (18) aufweist, an den das proximale Ende (4) des Hauptrohrs (2), nachdem es in die genannte Bohrung (15) hineingeführt wird, anlehnt.

5. Katheter nach Anspruch 2, dadurch gekennzeichnet, daß der Körper (9) der Betätigungsmittel (8) eines vieleckigen oder zylindrischen Querschnitts ist und an seinem Außenumkreis (21) Kanten (20) umfaßt, die Zeugenden gemäß angeordnet sind, um die Übertragung des vom Bedienungsmannes auf dem Katheter (1) ausgeübten Drehmoments zu erleichtern.

6. Katheter nach Ansprüchen 1 und 3, dadurch gekennzeichnet, daß die Anschlußmittel (7) des Universaltyps sind und aus einem Ansatzstück (23) bestehen, das aus einem zylindrischen Abschnitt (28) und einem kegelstumpfartigen Abschnitt (28A) gebildet ist, die sich in der gegenseitigen axialen Verlängerung befinden, wobei der zylindrische Abschnitt (28) an seinem freien Ende (24) und an seinem Außenumkreis (25) Finger (26) umfaßt, die dazu bestimmt sind, mit dem Gewinde der Anschlußmittel eines externen Geräts zusammenzuwirken und wobei ein zylindrischer Teil (30), der in der Lage ist, mit der Muffe (13) der Betätigungsmittel (8) zusammenzuwirken, an das spitzförmige Ende (29) des kegelstumpfartigen Abschnitts (28A) anschließt.

7. Katheter nach Ansprüchen 4 und 6, dadurch gekennzeichnet, daß die Bohrung (15) in der Muffe (13) im Bereich des Endes (19) dieser letzten innerhalb der Zentralbohrung (10) des Körpers (9) und jenseits des Innenumkreisrandes (18) einen Rücksatz (31) eines Durchmessers, der geringfügig größer als der Außendurchmesser (35) des zylindrischen Teils (30) ist, aufweist, um dessen Hineinführung zu erleichtern, wobei die Bohrung (15) außerdem an deren in die Zentralbohrung (10) ausmündenden Ende (32) mit einem inwendigen Schulter (33) versehen ist, der geeignet ist, mit einem gewissen Druck mit dem Außenumkreis (36) des genannten zylindrischen Teils (30) zusammenzuwirken und die Dichtigkeit zwischen den Betätigungsmitteln (8) und den Anschlußmitteln (7) zu sichern.

8. Katheter nach Ansprüchen 1 und 6, dadurch gekennzeichnet, daß die Drehmittel (37) aus einer Büchse (38) eines Außendurchmessers (39) gebildet ist, der geringfügig kleiner als der Innendurchmesser (30) der Zentralbohrung (10) des Körpers (9) ist, um deren Hineinführung in diese letzte zu erlauben, wobei die genannte Büchse (38), einerseits, mittels einer ringförmigen Seitenplatte (41), die sich an deren dem zylindrischen Abschnitt (28) zugewandten Ende (42) befindet, und, andererseits, über in deren Innenteil (44) angeordnete und mit dem kegelstumpfartigen Abschnitt (28A) zusammenwirkende Verstärkungsrippen (43) mit dem Ansatzstück (23) fest verbunden ist.

9. Katheter nach Anspruch 8, dadurch gekennzeichnet, daß die Büchse (38) einerseits Mittel (45) zum Festhalten der Anschlußmittel (7) in den Betätigungsmitteln (8) umfaßt, wobei diese Mittel (8) von einem Umkreiswulst (46), mit dem der Außenumkreis (47) im Bereich des Endes (42) der genannten Büchse (38) versehen ist, und der in eine in der Innenwand (40) des Körpers (9) hineingreift, und, andererseits, einem an dessen freien Ende (51) vorgesehenen Schulter (52), der mit einem gewissen Spiel mit der genannten Innenwand (50) des Körpers (9) zusammenwirkt, um zur Führung der Betätigungsmittel (8) während der Drehung bezüglich der Anschlußmittel (7) beizutragen, gebildet sind.
